(19) Europäisches Patentamt
European Patent Office
Office européen des brevets

(11) Publication number: 0 174 720 A1

(12) # EUROPEAN PATENT APPLICATION

(21) Application number: 85304926.0

(22) Date of filing: 10.07.85

(51) Int. Cl.$^4$: C 07 D 319/06

(30) Priority: 11.07.84 US 629642

(43) Date of publication of application: 19.03.86 Bulletin 86/12

(84) Designated Contracting States: AT BE CH DE FR GB IT LI LU NL SE

(71) Applicant: WARNER-LAMBERT COMPANY
201 Tabor Road
Morris Plains New Jersey 07950(US)

(72) Inventor: Nordin, Ivan C.
241 Waverly Road
Holland Michigan 49423(US)

(72) Inventor: Thomas, James A.
740 College Avenue
Holland Michigan 49423(US)

(74) Representative: Jones, Michael Raymond et al,
HASELTINE LAKE & CO. Hazlitt House 28 Southampton Buildings Chancery Lane
London WC2A 1AT(GB)

(54) **Synthesis of substituted 1,3-dioxanes.**

(57) The cyclization of alpha, gamma-dihydroxy-beta-amino-substituted compounds to produce chemical intermediates for the preparation of amino acids is described.

EP 0 174 720 A1

0174720

# SYNTHESIS OF SUBSTITUTED 1,3-DIOXANES

## BACKGROUND

Derivatives of 1,3-dioxanes, such as 5-amino-4-phenyl-2,2-dimethyl-1,3-dioxane, have utility in the preparation of optically active amino acids and alpha-methyl amino acids, eg, the antihypertensive drug alpha-methyl Dopa. Traditional methods of making such dioxane derivatives have involved low reaction yields and generally unworkable reaction schemes.

One well-known scheme involves

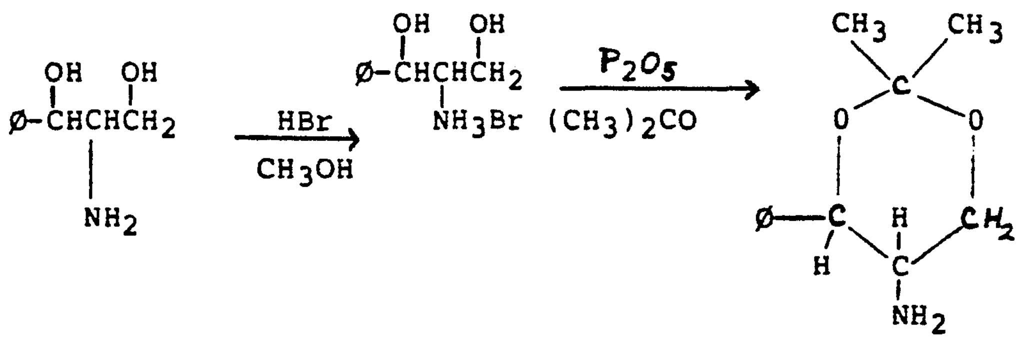


This reaction can not be efficiently scaled up due to large acetone requirements, problems with disposal of $H_3PO_4$ residues, and the need for purification of the product prior to further use.

*Chemical Abstracts* paragraphs 52:208i and 59:5175d, the disclosures of which are hereby incorporated by reference, illustrate this type of reaction.

## THE INVENTION

It has been discovered that the cyclization of alpha, gamma-dihydroxy beta-amino substituted compounds to yield 2,2 di-substituted, 5-amino-1,3-dioxanes can be carried out in a one pot reaction process involving three steps.

Generally, the process comprises the steps of:

(1) reacting at least one compound of formula I

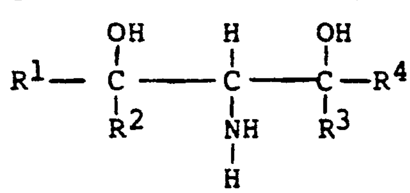

(I)

with at least one compound of formula II

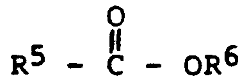

(II)

under suitable conditions to produce at least one compound of formula III

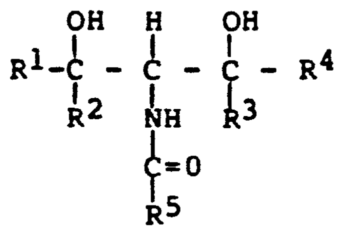

(III)

wherein $R^1$ through $R^5$ are independently selected from hydrogen and $C_{1-6}$ organic moieties, and $R^6$ is a $C_{1-6}$ alkyl or aryl moiety;

(2) reacting the product of step (1) under suitable conditions with at least one compound of formula IV

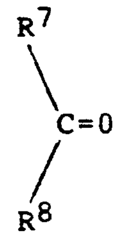

(IV)

in the presence of at least one compound of formula V

$$(R^9)_a - C - (OR^{10})_b \quad (V).$$

to produce at least one compound of formula VI

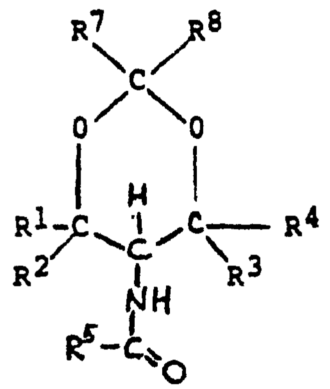

(VI)

wherein $R^7$, $R^8$ and $R^9$ and $R^{10}$ are the same as $R^6$, and the sum of a and b is 4; and
(3) contacting the product of step (2) with hydrazine under suitable conditions to yield at least one compound of formula VII

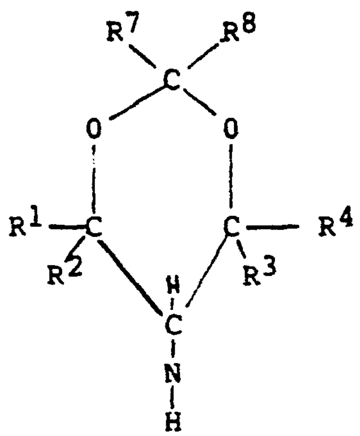

(VII).

Optionally, the products made using the above scheme can then be treated via the method disclosed in an article by K. Weinges, G. Graab, D. Nagel, and B. Stemmle, at Chem. Berichte, 104, 3594-3606 (1971) to yield pharmaceutically active materials, eg. alpha-methyl Dopa. The disclosure of that article is hereby incorporated by reference.

In a preferred embodiment, 1-phenyl, 2-aminopropane-1,3-diol is contacted with methyl formate in methanol solution to yield the N-formyl derivative

of the starting diol. To that derivative is added HBr, 2,2-dimethoxypropane, and acetone to produce 2,2-dimethyl, 4-phenyl, 5-formylamino-1,3-dioxane. Reaction of that product with hydrazine hydrate in water gives about a 78% yield of 5-amino-4-phenyl-2,2-dimethyl-1,3-dioxane.

The use of the N-acetyl protecting group instead of the N-formyl group gives yields of about 70%. However, the hydrazinolysis step took 60 or more hours to complete.

One aspect of the invention deals with the preparation of substituted 1,3-dioxanes.

Another aspect of the invention is concerned with producing chemical intermediates from which biologically- or pharmaceutically- active substances can be made.

ADVANTAGES

The process of the invention has several advantages over known processes for producing substituted 1,3-dioxanes of the types described herein.

Generally, the subject process is faster and simpler than known processes, but produces dioxanes in good yields. It is this speed, simplicity, and efficiency which results in relative ease of commercial scale-up. The process can be considered a onepot scheme, which results in products which are suitably pure for many purposes without distillation.

Other aspects and advantages of the invention can be ascertained from a consideration of the following description.

DESCRIPTION OF THE INVENTION

The invention involves the preparation of 1,3-dioxanes having chemical structures rendering them

useful per se or useful as intermediates in the preparation of other compounds, eg, antihypertensive substances, such as L-alpha-methyl Dopa.

The substituted 1,3-dioxanes made in accordance with the principal reactions described herein can be further reacted to yield biologically-active substances, eg., alpha-methyl Dopa or other alpha-methyl amino acids. Suitable reactions for the production of such substances are described by K. Weinges and B. Stemmle Chem. Berichte, 106, 2291-2297 (1973); K. Weinges, G. Graab, D. Nagel, and B. Stemmle, Chem. Berichte, 104, 3594-3606 (1971); and K. Weinges, G. Brune, and H. Driste, Annalen, 1980, 212-218, the disclosures of which are hereby incorporated by reference.

Stated broadly, the invention deals with a three step process for the cyclization of substituted alpha-,gamma-dihydroxy,-beta-amino compounds.

The three steps can be represented as follows:

Step 1

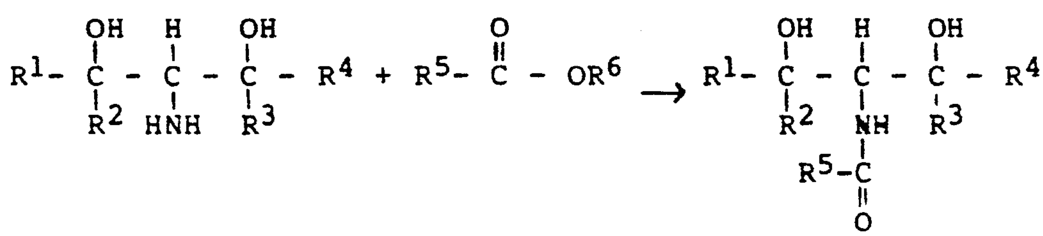

Step 2

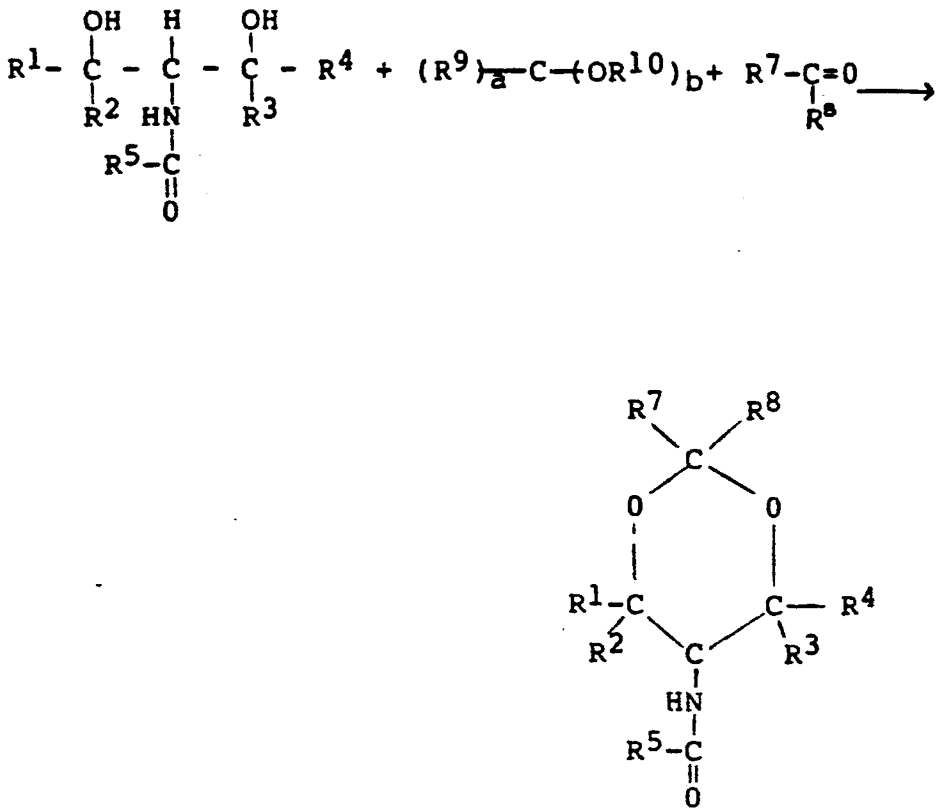

Step 3

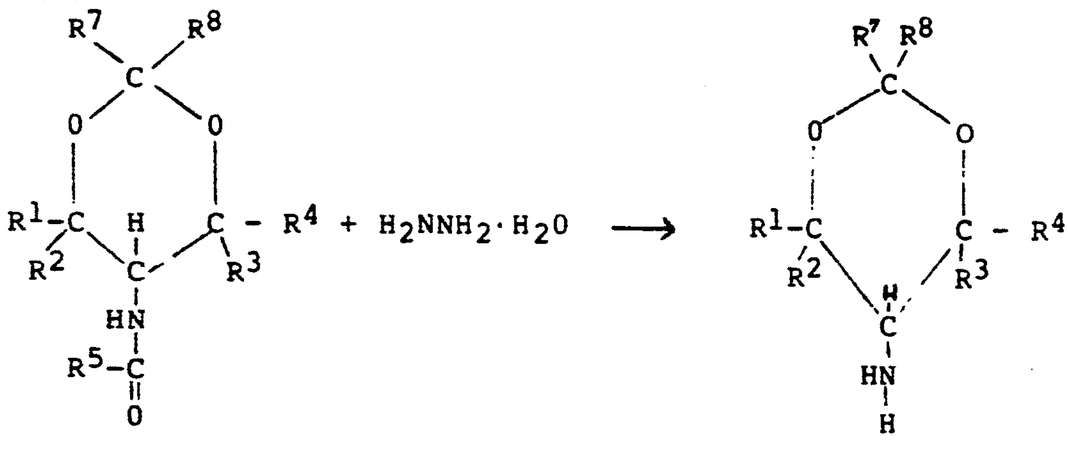

In these equations, $R^1$ through $R^5$ are independently selected from H, $C_{1-6}$ alkyl, and aryl moieties and $R^6$ through $R^{10}$ are $C_{1-6}$ alkyl groups, preferably methyl groups.

In these reactions, $R^1$, $R^2$, $R^3$, $R^4$, and $R^5$ are each, independently a hydrogen or a $C_{1-6}$ organic moiety. Without being limited thereto, the organic moiety may be a $C_{1-6}$, straight or branched, alkyl radical (optionally substituted); or an aryl radical, which may be mono- or bi- cyclic and which may be substituted or unsubstituted for example substituted or unsubstituted benzyl or naphthyl, preferably substituted or unsubstituted benzyl. $R^6$ is chosen from $C_{1-6}$ alkyl, straight or branched, moieties, $C_{1-6}$ alkanoyl, straight or branched moieties and aryl moieties. These alkyl, alkanoyl and aryl moieties may optionally be substituted and the aryl radical may be a substituted or unsubstituted benzyl or naphthyl radical. Preferably $R^6$ is a methyl radical or an acetyl radical, most preferably a methyl radical.

$R^7$, $R^8$, $R^9$, and $R^{10}$ are chosen from $C_{1-6}$, straight or branched, alkyl radicals (which may optionally be substituted) and aryl moieties (mono- or bi-cyclic, unsubstituted or substituted).

Suitable amine reactants for step 1 comprise alpha, gamma-dihydroxy-beta-amino compounds of the formula shown above. Further substituted reactants, such as 1-phenyl-,2-amino,1,3-propane diol are also operable. Mixtures of such compounds can be used.

Step 1 involves the introduction into the amine molecule of a group which shields or protects the nitrogen atom therein from undesireable side reactions. Typically, protecting groups are carbonyl-containing substances and other reagents whose reaction with the nitrogen atom leave N-C linkages which are not readily attached during subsequent intermediate reactions in

the cyclization scheme. The protecting groups should be removable by hydrolysis or other suitable reaction, following production of the desired 1,3-dioxanes.

Thus, the other class of reactants useful in step 1 are typically carbonyl-containing compounds which supply protecting groups. Generally, they are anhydrides, esters, or their functional equivalents. Useful carbonyl-containing reactants include alkyl acylates, acetic anhydride and the like. Carboxyl-containing compounds are preferred. One highly preferred carboxyl-containing reactant is methyl formate. Mixtures of such reactants are operable.

It is generally preferred that step 1 be carried out in the presence of at least one diluent. While aromatic diluents, e.g., toluene, and combinations of aromatics with hydroxyl-containing diluents are operable, it is highly preferred that $C_{1-8}$ alkanols, such as ethanol, propanol, octanol, and the like, and mixtures thereof, be employed. Methanol is particularly preferred.

The product of step 1--ie., the N-acyl derivative of the dihydroxy amino reactant for step 1--is then contacted with at least two reagents. One is at least one carbonyl containing species. The other is at least one ketal or acetal species.

The carbonyl-containing species useful in step 2 generally supplies the carbon atom in the number 2 position of the 1,3-dioxanes--i.e., it is the source of the protecting group. Preferred species include one or more of the aldehydes and ketones conforming to the general formula given above. Suitable carbonylcontaining substances include cyclic aldehydes and ketones, eg., non-cyclic species, eg., dimethyl ketone, and the like, and mixtures thereof. Dimethyl ketone is highly preferred.

The other reactant(s) used in step 2 generally comprise at least one compound of the general formula $(R^9)_aC(OR^{10})_b$. Useful compounds include those reagents useful as water scavengers, which reagents do not cause deleterious side reactions. 2,2-dimethoxypropane is a highly preferred reagent. Mixtures of such reagents are operable.

The product of step 2--ie., the 5-N-acyl-substituted 2,2-di-substituted-1,3-dioxane--is then contacted, in step 3, with hydrazine or other suitable reagent to remove the N-substituent and leave an amino group. Other suitable agents and combinations of agents include hydrolyzing agent(s) such as KOH,$H_2O$; NaOH,$H_2O$; KOH, ethylene glycol; and the like, and mixtures thereof.

When hydrazine is used, it is generally preferred that it be in a hydrated form.

<u>REACTION CONDITIONS</u>

The conditions under which reaction steps 1,2, and 3 should be carried out can be derived from various considerations, such as the type of reaction vessel, properties of the reactants, etc.

Generally, reaction temperatures for the three

steps will conform to the parameters given in the following table:

| | Temperature Range (°C) | |
|---|---|---|
| Step | Broad | Preferred |
| 1 | -20 - 50 | 10 - 30 |
| 2 | -20 - 50 | 5 - 30 |
| 3 | 50 - 150 | 80 - 120 |

Reasonable extrapolation from these temperatures, as well as the use of suitable pressures, etc. are within the purview of the skilled artisan.

SUBSEQUENT REACTIONS

The product of step 3 is an amino-substituted 1,3-dioxane. Such compounds are known to be intermediates in the synthesis of such compounds as optically active amino acids and alpha-methyl amino acids. Alpha-methyl Dopa,

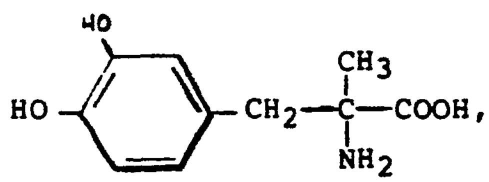

is one pharmaceutically active compound which can be made using the products of the instant process as intermediates. The production of alpha-methyl-Dopa can be carried out under suitable conditions via the processes discussed above.

Suitable recovery techniques, such as extraction and distillation, can be used during and/or after the three steps outlined herein.

The invention can be better understood from a consideration of the following example.

EXAMPLE

All solvents and reagents are commercially obtained with no further purification. 2-Amino-1-phenyl-1,3-propanediol was obtained from Quimica Sintetica, S.A., Madrid.

To a 250 ml flask was added 5.0g (0.0299 mol) of 1-phenyl-2-amino-1,3-propane diol, 25 ml of methanol, and 19 ml (0.308 mol) of methyl formate. The mixture was stirred for 3.5 hrs, then the solvent was evaporated. To the crystalline residue was added 75 ml of acetone, 8 ml of 2,2-dimethoxypropane, and 2.0 ml of 1.0 M HBr (in methanol). This mixture was stirred in an ice water bath for 2.5 hours and the solvents were evaporated.

To the viscous residue was added 25 ml of 85% $H_2NNH_2$ $H_2O$ and the mixture was refluxed for 1 hr. The cooled mixture was extracted with 3-25 ml portions of toluene. The toluene phase was washed with 10 ml of $H_2O$, dried ($Na_2SO_4$) and evaporated to give 4.8 g (78%) of a yellow oil, 5-amino-4-phenyl-2,2-dimethyl-1,3-dioxane.

Reasonable variations, such as those which would occur to a skilled artisan, can be made herein without departing from the scope of the invention.

CLAIMS:

1. A process for the preparation of substituted derivatives of 1,3-dioxane from alpha-, gamma- dihydroxy-, beta-amino-substituted compounds comprising the steps of

(1) reacting a compound of the general formula I

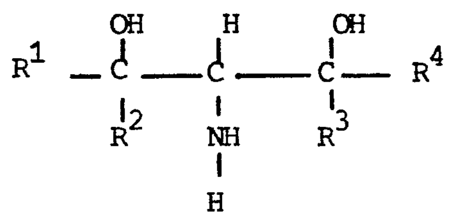

(I)

under suitable conditions with a compound of the general formula II

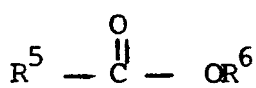

(II)

to produce a compound of the general formula III

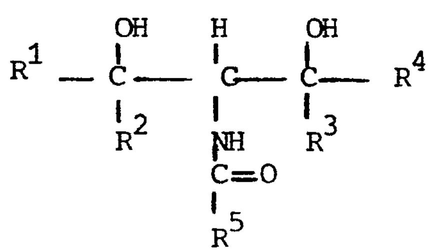

(III)

wherein $R^1$, $R^2$, $R^3$, $R^4$ and $R^5$ are each, independently a hydrogen or a $C_{1-6}$ organic moeity and R is a $C_{1-6}$ alkyl moiety, a $C_{1-6}$ alkanoyl moiety or aryl moiety

(2) reacting the product of step (1) under suitable conditions with at least one compound of the general formula IV

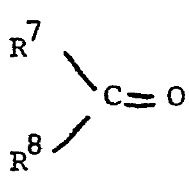

(IV)

in the presence of a compound of the general formula V

$$(R^9)_a — C — (OR^{10})_b \qquad (V)$$

to produce a compound of the general compound VI

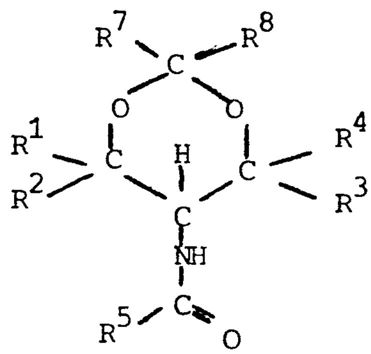

(VI)

wherein $R^7$, $R^8$, $R^9$, and $R^{10}$ are each, independently, a $C_{1-6}$ alkyl moiety or an aryl moiety and a and b are chosen from 0, 1, 2, 3 and 4, the sum of a and b being equal to 4;

(3) contacting the product of step (2) with a hydrolysing agent under suitable conditions to yield a compound of formula VII

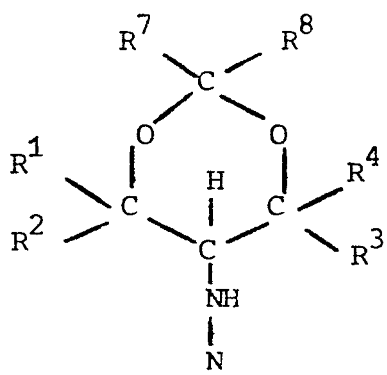

(VII)

2. The process of Claim 1 wherein step 2 takes place under acid conditions.

3. A process according to Claim 1 or 2, wherein $R^7$ and $R^8$ are both methyl groups.

4. A process according to Claim 1, 2 or 3,

wherein $R^9$ and $R^{10}$ are both methyl groups.

5. A process according to any preceding claim, wherein $R^5$ is a hydrogen.

6. A process according to any one of Claims 1 to 4 wherein $R^5$ is a methyl group.

7. A process according to any preceding claim, wherein step 1 takes place in an alcohol solvent.

8. A process according to any one of Claims 1 to 6, wherein step 1 takes place in the presence of a diluent.

9. A process according to any preceding claim, wherein the compound of formula VII is 5-amino-4-phenyl-2,2dimethyl-1,3-dioxane.

10. A process according to any preceding claim, wherein the hydrolysing agent in step III is hydrazine.

11. A process according to any preceding claim, wherein the compound of formula VII is further reacted to produce a pharmaceutically active material.

AMENDED CLAIMS



wherein $R^9$ and $R^{10}$ are both methyl groups.

5. A process according to any preceding claim, wherein $R^5$ is a hydrogen.

6. A process according to any one of Claims 1 to 4 wherein $R^5$ is a methyl group.

7. A process according to any preceding claim, wherein step 1 takes place in an alcohol solvent.

8. A process according to any one of Claims 1 to 6, wherein step 1 takes place in the presence of a diluent.

9. A process according to any preceding claim, wherein the compound of formula VII is 5-amino-4-phenyl-2,2dimethyl-1,3-dioxane.

10. A process according to any preceding claim, wherein the hydrolysing agent in step III is hydrazine.

11. A process for preparing alpha-methyl Dopa which process comprises preparing 5-amino-4-phenyl-2,2-dimethyl-1,3-dioxane by a process according to Claim 9 and converting the thus formed 1,3-dioxane derivative to alpha-methyl Dopa.

0174720

European Patent Office

## PARTIAL EUROPEAN SEARCH REPORT

which under Rule 45 of the European Patent Convention shall be considered, for the purposes of subsequent proceedings, as the European search report

Application number: EP 85304926.0

### DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | CHEMICAL ABSTRACTS, vol. 59, no. 5, September 2, 1963, Columbus, Ohio, USA<br>FUJISAWA PHARMACEUTICAL CO. LTD. "5-Amino-6-phenyl-1,3-dioxane derivatives" column 5175, abstract-no. 5175d<br>& JAPAN. 7977. (62) and JAPAN. 7978 (62), July 11, 1959<br>-- | 1 | C 07 D 319/06 |
| A | CHEMICAL ABSTRACTS, vol. 92, no. 21, May 26, 1980, Columbus, Ohio, USA<br>K. WEINGES, K.P. KLOTZ, H. DROSTE "Asymmetric syntheses.V.Optically active 5-ammino-4-phenyl-1,3-dioxanes and their effect on the stereo selectivity of the asymmetrical Strecker synthesis" page 596, column 2, abstract-no. 180 582x<br>& Chem. Ber. 1980, 113(2), 710-21<br>-- | 1 | TECHNICAL FIELDS SEARCHED (Int. Cl.4)<br>C 07 D 319/00 |

### INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-10

Claims searched incompletely: –

Claims not searched: 11

Reason for the limitation of the search:

The subject of the claim is not clearly defined

| Place of search | Date of completion of the search | Examiner |
|---|---|---|
| VIENNA | 17-10-1985 | BRUS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1505.1 03.82

European Patent Office

# PARTIAL EUROPEAN SEARCH REPORT

0174720

Application number

EP 85304926.0

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| A | EP - A1 - 0 068 709 (STAUFFER CHEMICAL COMPANY)<br>* Claim 1 *<br>---- | 1 | |
| | | | TECHNICAL FIELDS SEARCHED (Int. Cl.4) |

EPO Form 1505.3 06.78